(19) Europäisches Patentamt
European Patent Office
Office européen des brevets

(11) EP 0 670 342 B1

(12) **EUROPÄISCHE PATENTSCHRIFT**

(45) Veröffentlichungstag und Bekanntmachung des Hinweises auf die Patenterteilung:
**15.04.1998 Patentblatt 1998/16**

(51) Int Cl.$^6$: **C08G 77/46**, A61K 7/06

(21) Anmeldenummer: **95102242.5**

(22) Anmeldetag: **18.02.1995**

(54) **Polysiloxan-Polyoxyalkylen-Blockmischpolymerisate und ihre Verwendung als Zusatzmittel für Haarkosmetika**

Polysiloxane-polyoxyalkylene block copolymers and their use as cosmetic additive for hair

Copolymères séquencés de polysiloxane et polyoxyalkylène et leur usage comme additif dans des produits capillaires

(84) Benannte Vertragsstaaten:
**AT BE DE DK ES FR GB IT NL**

(30) Priorität: **04.03.1994 DE 4407189**

(43) Veröffentlichungstag der Anmeldung:
**06.09.1995 Patentblatt 1995/36**

(73) Patentinhaber: **Th. Goldschmidt AG**
**45127 Essen (DE)**

(72) Erfinder:
- **Burkhart, Georg, Dr.**
  **D-45239 Essen (DE)**
- **Langenhagen, Rolf-Dieter**
  **D-45529 Hattingen (DE)**
- **Weier, Andreas, Dr.**
  **D-45289 Essen (DE)**
- **Zellmer, Volker, Dr.**
  **D-46240 Bottrop (DE)**

(56) Entgegenhaltungen:
**EP-A- 0 381 318**
**EP-A- 0 449 050**
**EP-A- 0 499 051**
**EP-A- 0 501 791**
**EP-A- 0 558 990**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Polysiloxan-Polyoxyalkylen-Blockmischpolymerisate und ihre Verwendung als Zusatzmittel für Haarkosmetika, insbesondere für Haarshampoos.

Polyoxyalkylen-Polysiloxan-Blockmischpolymerisate (im folgenden als Polyethersiloxane bezeichnet) haben einen weiten Anwendungsbereich gefunden. Man kann sie als Tenside, Emulgiermittel, Dispergiermittel, Lackverlaufsmittel, Schmiermittel, als Hilfsmittel bei der tertiären Erdölförderung, als Schaumstabilisatoren bei der Polyurethanverschäumung, als Textilhilfsmittel zum Avivieren von Fasern, Garnen oder flächigen Textilprodukten und zur Hydrophilierung von sanitären Erzeugnissen aus Zellulosefasern und für viele andere Zwecke einsetzen.

Die Polyethersiloxane sind deshalb so vielseitig brauchbar, da man ihre Eigenschaften, insbesondere ihre Hydrophil/Hydrophob-Balance, durch geeignete Wahl des Siloxanblockes oder der Siloxanblöcke einerseits und durch geeigneten Aufbau des Polyetherblockes oder der Polyetherblöcke beeinflussen und auf den gewünschten Wert bringen kann.

So kann der Siloxanblock linear oder verzweigt sein, wobei die absolute Anzahl der difunktionellen und trifunktionellen Siloxy-Einheiten und ihr Zahlenverhältnis zueinander in weiten Grenzen schwanken kann. Es ist ferner möglich, außer den Polyetherblöcken andere modifizierende Gruppen an ein Si-Atom zu binden. Beispiele solcher Reste sind langkettige Kohlenwasserstoffreste mit bis zu 30 Kohlenstoffatomen, mit Halogenatomen, Cyanogruppen oder polaren Resten substituierte Kohlenwassenstoffreste, Hydroxylgruppen, etc.

Ebenso können die Polyetherblöcke unterschiedlichen Aufbau haben. Jeder Polyoxyalkylenblock kann aus verschiedenen Oxyalkyleneinheiten, vornehmlich aus Oxyethylen-, Oxypropylen- und Oxybutylen-Einheiten zusammengesetzt sein. Dabei kann das Gewichtsverhältnis dieser Einheiten zueinander sowie das Molgewicht des Polyoxyalkylenblockes variiert werden. Von Bedeutung ist auch die Endgruppe des Polyoxyalkylenblockes, die reaktiv (z. B. OH-Gruppe) oder inert (z. B. Alkoxy-Gruppe) sein kann.

Der Polyoxyalkylenblock kann mit dem Polysiloxanblock durch eine hydrolytisch stabile C-Si-Bindung oder die hydrolytisch weniger stabile C-O-Si-Bindung verknüpft sein.

Es ist auch möglich und bei vielen Anwendungszwecken erwünscht, unterschiedliche Polyetherblöcke an den Polysiloxanblock zu binden. Dabei unterscheiden sich die unterschiedlichen Polyetherblöcke in bezug auf ihr Molgewicht und/oder ihre Hydrophilie und/oder ihre inerten oder reaktiven Endgruppen.

Polysiloxane mit unterschiedlichen Polyetherblöcken sind unter anderem in folgenden Patentschriften und veröffentlichten Patentanmeldungen beschrieben:

DE-PS 15 70 647: Chlorpolysiloxanylsulfate werden mit Gemischen von Alkylenoxid-Addukten umgesetzt, welche aus 50 bis 95 OH-Äquivalentprozent Polyalkylenglykolmonoethern, die aus Ethylenoxid- und Propylenoxideinheiten bestehen und einen Gehalt von 40 bis 70 Gewichtsprozent Oxypropyleneinheiten und ein Molgewicht von 1000 bis 3000 aufweisen, deren Hydroxylgruppen vorzugsweise sekundär sind, und 5 bis 50 OH-Äquivalentprozent Alkylenoxid-Addukten mehrwertiger Hydroxylverbindungen eines Molgewichtes von 130 bis 3500, deren Polyalkylenglykolkomponente aus Ethylenoxid- und/oder Propylenoxideinheiten bestehen und die ein OH-Äquivalentgewicht bis zu 1750 haben und deren Hydroxylgruppen vorzugsweise sekundär sind, bestehen und wobei die Mengenverhältnisse so gewählt sind, daß auf ein Säureäquivalent des Chlorpolysiloxanylsulfates höchstens 1,4, vorzugsweise 1,05 bis 1,2, OH-Äquivalente kommen.

DE-PS 16 94 366: Sie betrifft Polysiloxan-Polyoxyalkylen-Blockmischpolymerisate, deren Polysiloxanblock in an sich bekannter Weise aufgebaut ist, deren Polyoxyalkylenblock jedoch aus

25 bis 70 Gewichtsprozent eines Polyoxyalkylens mit einem durchschnittlichen Molekulargewicht von 1600 bis 4000 und einem Ethylenoxidgehalt von 20 bis 100 Gewichtsprozent, Rest Propylenoxid und gegebenenfalls höhere Alkylenoxide, und

30 bis 75 Gewichtsprozent eines Polyoxyalkylens mit einem durchschnittlichen Molekulargewicht von 400 bis 1200 und einem Ethylenoxidgehalt von 65 bis 100 Gewichtsprozent, Rest Propylenoxid und gegebenenfalls höhere Alkylenoxide, besteht.

DE-OS 25 41 865: Die Polysiloxan-Polyoxyalkylen-Blockmischpolymerisate sind bezüglich ihrer Polyoxyalkylenblöcke so definiert, daß der eine Polyoxyalkylenblock ein mittleres Molgewicht von 900 bis 1300 hat und zu 30 bis 55 Gew.-% aus Ethylenoxid, Rest Propylenoxid, und der andere Polyoxyalkylenblock ein mittleres Molgewicht von 3800 bis 5000 hat und zu 30 bis 50 Gew.-% aus Ethylenoxid, Rest Propylenoxid besteht.

EP-OS 0 275 563: Das in dieser veröffentlichten europäischen Patentanmeldung beschriebene Blockmischpolymerisat umfaßt drei verschiedene Polyoxyalkylenblöcke, nämlich einen Block, welcher 20 bis 60 Gew.-% Oxyethyleneinheiten enthält, bei einem Molgewicht von 3000 bis 5500, einen weiteren Block mit 20 bis 60 Gew.-% Oxyethyleneinheiten und einem Molgewicht von 800 bis 2900 und einen dritten Block nur aus Polyoxypropyleneinheiten und einem Molgewicht von 130 bis 1200.

Die Herstellung derartiger Polyethersiloxane kann entsprechend dem Stand der Technik auf verschiedene Weisen erfolgen. Hierzu wird auf die folgenden Patentschriften verwiesen:

DE-PS 10 12 602: Polyethermono- oder -diole werden in Gegenwart eines Lösungsmittels und eines Katalysators mit Polysiloxanen umgesetzt, welche endständig ≡Si-OAlkylgruppen aufweisen, wobei der freigesetzte aliphatische Alkohol aus dem Reaktionsgemisch abdestilliert wird. Es handelt sich somit um eine Umesterung.

DE-PS 17 95 557: Äquilibrierte Siloxangemische der allgemeinen Formel $R_xSiO_y(SO_4)_zX_{4-(z+2x+2y)}$ (R beliebige einwertige Kohlenwasserstoffreste; X Halogen- oder Alkoxyreste; x = 0,9 bis 2,2; y = 0,75 bis 1,75; z = 0,0001 bis 0,5; 4 > (x+2y+2x) > 2) werden mit Polyethermonoolen umgesetzt, die bei der Reaktion freiwerdende Säure neutralisiert, das Reaktionsprodukt filtriert und vom Lösungsmittel befreit.

Bei beiden Verfahren werden Polyethersiloxane erhalten, bei denen die Polyethergruppe(n) mit dem Siloxangerüst über eine Si-O-C-Brücke verbunden ist (sind).

Die Herstellung von Polyethersiloxanen, bei denen die Polyethergruppe(n) mit dem Siloxangerüst über eine Si-C-Brücke verbunden ist (sind), kann der DE-PS 31 33 869 entnommen werden. Nach der dort beschriebenen Verfahrensweise werden Allylpolyether mit Polysiloxanen, welche Si-H Gruppen aufweisen, in Gegenwart von speziellen Platinkatalysatoren und gegebenenfalls in Gegenwart von inerten Lösungsmitteln umgesetzt.

Die Erfindung befaßt sich mit dem technischen Problem, Polyethersiloxane aufzufinden, welche in besonderer Weise für den Einsatz in der Haarkosmetik geeignet sind und insbesondere als Zusatzmittel für Haarshampoos eingesetzt werden können. Dabei wird angestrebt, den Griff und die Kämmbarkeit des Haares zu verbessern.

Ein Gegenstand vorliegender Erfindung sind somit neue Poly-siloxan-Polyoxyalkylen-Blockmischpolymerisate der allgemeinen Formel

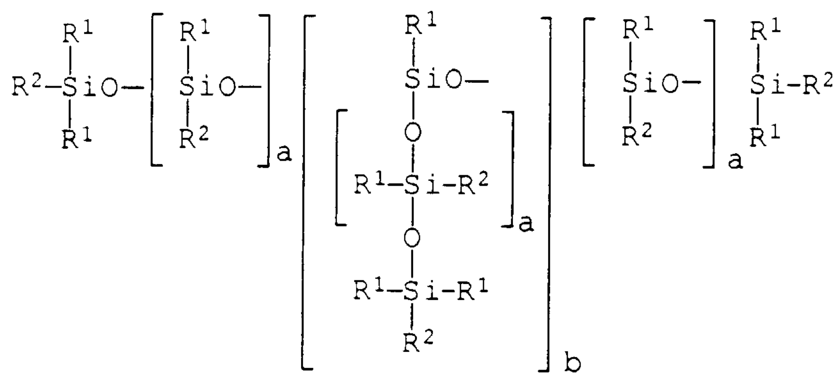

wobei die Reste

$R^1$ Alkylreste mit 1 bis 4 Kohlenstoffatomen oder Phenylreste sind, jedoch mindestens 90 % der Reste $R^1$ Methylreste sind,

$R^2$
(1) den Resten $R^1$ entsprechen oder
(2) durch Addition von Kohlenwasserstoffen mit einer olefinischen Doppelbindung an SiH-Gruppen des Siloxans erhaltene Reste mit 6 bis 30 Kohlenstoffatomen oder
(3) $-M-R^3$ Reste sind, wobei

M ein zweiwertiger Rest der Formel $-R^4_xO-$ ist, in dem $R^4$ ein zweiwertiger Alkylenrest, der auch verzweigt sein kann, ist und x einen Wert von 0 oder 1 hat,

$R^3$ ein Gemisch von Polyetherresten, enthaltend mindestens einen

(1) Polyoxyalkylenrest A mit einem mittleren Molgewicht von 600 bis 5500, der aus 20 bis 100 Gew.-% Oxyethyleneinheiten und 80 bis 0 Gew.-% Oxypropyleneinheiten besteht, und einen

(2) Polyoxyalkylenrest B mit einem mittleren Molgewicht von 700 bis 5000, der aus 0 bis < 20 Gew.-% Oxyethyleneinheiten und 100 bis 80 Gew.-% Oxypropyleneinheiten besteht,

wobei jeweils bis zu 20 Gew.-% der Oxypropyleneinheiten durch Oxybutyleneinheiten ersetzt sein können, und
wobei das Molverhältnis der Polyoxyalkylenreste A zu den Polyoxyalkylenresten B 1 : 4 bis 4 : 1 beträgt,

mit der Maßgabe, daß

(I) die Anzahl der Reste $R^2$ mit der Bedeutung (2) mindestens gleich 1 und höchstens 30% des Zahlenwertes der Anzahl der Siliciumatome ist und

(II) im durchschnittlichen Blockmischpolymerisat mindestens zwei Reste $R^3$ vorhanden sind,

b einen Wert von 0 bis 10 hat,
a einen Wert von 10 bis 100 hat, wenn b = 0 ist oder einen Wert von 3 bis 70 hat, wenn b > 0 und ≤ 4 ist, oder einen Wert von 3 bis 30 hat, wenn b > 4 ist.

Ein wesentliches Merkmal der vorliegenden Erfindung besteht in der gleichzeitigen Anwesenheit der Kohlenwasserstoffreste mit 6 bis 30 Kohlenstoffatomen und der mit dem Polysiloxangerüst verbundenen Polyoxyalkylenreste sowie ihrer Art und Menge.

Der Rest $R^1$ ist vorzugsweise ein Methylrest.

$R^2$ entspricht dabei in der überwiegenden Anzahl der Reste den Resten $R^1$. Es ist jedoch ein erfindungswesentliches Merkmal, daß einige der Reste $R^2$ durch Addition von Kohlenwasserstoffen mit einer olefinischen Doppelbindung an SiH-Gruppen des Siloxans erhaltene Reste mit 6 bis 30 Kohlenstoffatomen sind.

Bevorzugte Beispiele solcher Kohlenwasserstoffreste sind die folgenden Reste

$-(CH_2)_{5-29}-CH_3$ 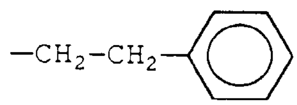 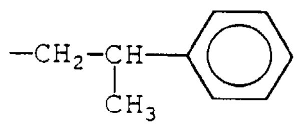

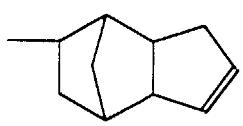 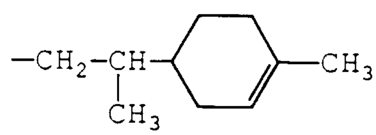

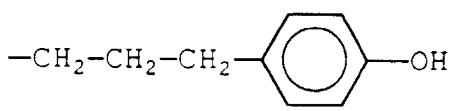 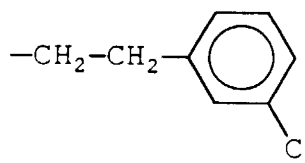

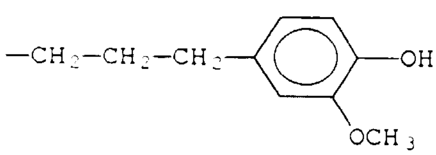 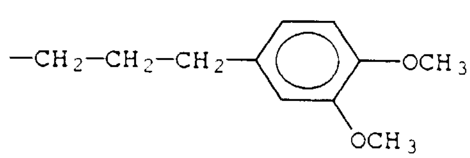

Die Reste $R^2$ können ferner die Bedeutung von Resten der Formel $-R^4{}_xO-$ haben.

Der Rest $R^4$ ist vorzugsweise ein $-(CH_2)_2-$ oder $-(CH_2)_3-$ Rest.

Vorzugsweise entsprechen die Polyoxyalkylenblöcke der Formel $(C_mH_{2m}O-)_nR^5$ , wobei die Indices n und m so gewählt sind, daß die Bedingungen hinsichtlich der Zusammensetzung und des jeweilgen Molgewichtes der verschiedenen Polyoxyalkylenblöcke erfüllt sind, und $R^5$ ein Alkylrest mit 1 bis 4 Kohlenstoffatomen, ein Acylrest oder ein $-O-CO-NH-R^6$ Rest, wobei $R^6$ ein Alkyl- oder Arylrest ist, ist.

Die Polyoxyalkylenreste A und B weisen unabhängig voneinander vorzugsweise ein mittleres Molgewicht von 1000 bis 4000 auf.

Die Blöcke A und B können mehrfach enthalten und an ein gemeinsames durchschnittliches Molekül gebunden sein. Für den Fall, daß die Polyoxyalkylenreste A und B mehrfach enthalten sind, müssen die Polyoxyalkylenreste der einzelnen Blocktypen nicht miteinander identisch sein. Die Polyetherreste A und B müssen nur den jeweiligen Bedingungen für die einzelnen Blocktypen entsprechen.

Vorzugsweise beträgt das Molverhältnis der Polyoxyalkylenreste A zu den Polyoxyalkylenresten B 1 : 3 bis 3 : 1.

Die erfindungsgemäßen Blockmischpolymerisate können nach an sich bekannten Verfahren hergestellt werden. Dabei ist zu unterscheiden, ob die Polyetherblöcke durch Si-C- oder Si-O-C- Bindungen mit dem Polysiloxan verbunden sind.

Zur Herstellung der erfindungsgemäßen Verbindungen mit Si-C- Verknüpfung ist das folgende Verfahren besonders bevorzugt:
Es werden an Organopolysiloxane der allgemeinen Formel

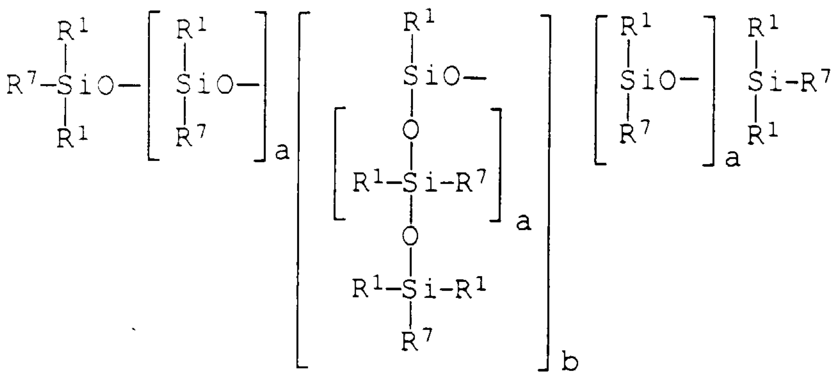

wobei die Reste

$R^1$ und die Indices a und b die bereits angegebene Bedeutung haben,
$R^7$ (1) den Resten $R^1$ entsprechen oder
(2) SiH-Reste sind,

mit der Maßgabe, daß mindestens 3 Reste $R^7$ SiH-Reste sind,

a) Kohlenwasserstoffe mit 6 bis 30 Kohlenstoffatomen und einer olefinischen Doppelbindung und

b) ein Gemisch von Polyethern der Formel

$$CH_2=CH-R^8-A \text{ und } CH_2=CH-R^8-B$$

wobei der Rest $R^8$ dem um die Gruppe $-(CH_2)_2-$ verminderten Rest $R^4$ entspricht und die Polyether im Molverhältnis 1 : 4 bis 4 : 1 vorliegen,

in Gegenwart von für die Hydrosilylierung geeigneten und hierfür bekannten Platinkatalysatoren, wie Hexachloroplatinsäure, nacheinander oder gleichzeitig hydrosilylierend addiert.

Man kann also zunächst die olefinischen Kohlenwasserstoffe an das Wasserstoffsiloxan addieren und dann das Polyethergemisch an die verbliebenen SiH-Gruppen addieren oder simultan beide Additionsreaktionen gleichzeitig vornehmen.

Ein weiteres bevorzugtes Verfahren betrifft die Herstellung der erfindungsgemäßen Verbindungen mit Si-O-C Bindungen. Dieses Verfahren ist dadurch gekennzeichnet, daß man Organopolysiloxane der allgemeinen Formel

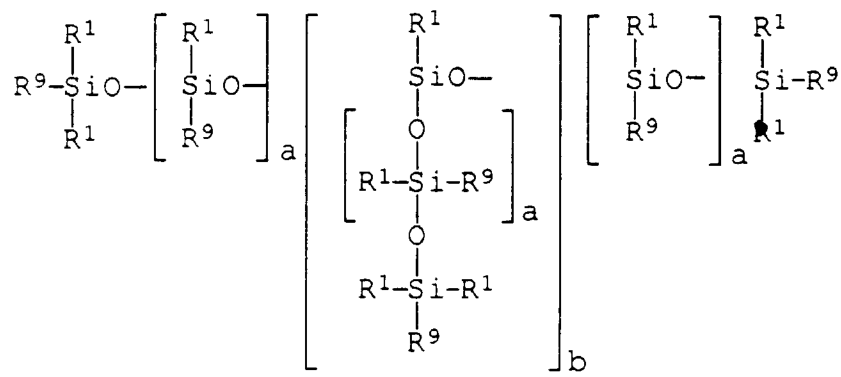

wobei die Reste

$R^1$ und die Indices a und b die bereits angegebene Bedeutung haben,

$R^9$
(1) den Resten $R^1$ entsprechen oder
(2) durch Addition von Kohlenwasserstoffen mit einer olefinischen Doppelbindung an SiH-Gruppen des Siloxans erhaltene Reste mit 6 bis 30 Kohlenstoffatomen oder
(3) Halogen- oder sonstige saure Reste, wie $-SO_{4/2}$ oder $-CH_3SO_3$ Reste sind,

mit der Maßgabe, daß mindestens 1 Rest $R^9$ die Bedeutung (2) und mindestens 2 Reste $R^9$ die Bedeutung (3) haben,

mit einem Gemisch der Polyether

A—OH und B—OH ,

welche in einem Molverhältnis von 1 : 4 bis 4 : 1 vorliegen, mit einer solchen Menge des Polyethergemisches A-OH und B-OH, daß im gewünschten Organopolysiloxan mindestens 2 Polyetherreste gebunden sind, in Gegenwart eines Säureacceptors bei Temperaturen von $\geq 50°C$ umsetzt.

Geeignete Säureacceptoren sind Ammoniakgas, Triethylamin und i-Propylamin.

Die erfindungsgemäßen Verbindungen zeigen die gewünschten Eigenschaften als Zusatzmittel für Haarkosmetika. Es ist deshalb ein weiterer Gegenstand der Erfindung, die erfindungsgemäßen Verbindungen als Zusatzmittel für Haarkosmetika, insbesondere für Haarshampoos, in Mengen von 0,5 bis 4 Gew.-%, bezogen auf Gesamtmenge des Shampoos, zur Verbesserung des Griffes und der Kämmbarkeit der Haare zu verwenden.

Bei den folgenden Beispielen werden nachstehende Verbindungen eingesetzt:

Siloxane:

I) $(CH_3)_3SiO-[(CH_3)_2SiO-]_{58}[(CH_3)HSiO-]_9Si(CH_3)_3$
II) $H(CH_3)_2SiO-[(CH_3)_2SiO-]_{80}[(CH_3)HSiO-]_8Si(CH_3)_2H$
III) $(CH_3)_3SiO-[(CH_3)_2SiO-]_{130}[(CH_3)HSiO-]_{18}Si(CH_3)_3$
IV) $(CH_3)_3SiO-[(CH_3)_2SiO-]_{100}[(CH_3)HSiO-]_{12}Si(CH_3)_3$
V)

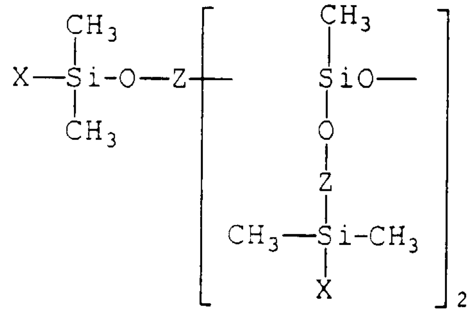

wobei

Z = $-[(CH_3)_2SiO-]_7[(CH_3)(C_8H_{17})SiO-]$ ist und
X = 75 % Cl und 25 % $SO_{4/2}$ bedeutet.

Polyether vom Typ A:

a) $CH_2=CH-CH_2O-(C_2H_4O-)_{12}CH_3$
b) $CH_2=CH-CH_2O-(C_2H_4O-)_{12}H$
c) $CH_2=CH-CH_2O-(C_2H_4O-)_{21}CH_3$
d) $C_4H_9O-(C_2H_4O-)_{12}H$
e) $C_4H_9O-(C_2H_4O-)_{82}(C_3H_6O-)_{5,4}H$
f) $CH_2=CH-CH_2O-(C_2H_4O-)_{45}(C_3H_6O-)_{34}CH_3$
g) $CH_2=CH-CH_2O-(C_2H_4O-)_{42}(C_3H_6O-)_{34}COCH_3$
h) $CH_2=CH-CH_2O-(C_2H_4O-)_{46}(C_3H_6O-)_{16}CH_3$
i) $CH_2=CH-CH_2O-(C_2H_4O-)_{16}(C_3H_6O-)_{12}CH_3$
k) $CH_2=CH-CH_2O-(C_2H_4O-)_{45}(C_3H_6O-)_{34}H$
l) $C_4H_9O-(C_2H_4O-)_{23}(C_3H_6O-)_{33}H$

Polyether vom Typ B:

m) $CH_2=CH-CH_2O-(C_2H_4O-)_5(C_3H_6O-)_{21}CH_3$
n) $CH_2=CH-CH_2O-(C_2H_4O-)_2(C_3H_6O-)_{32}CH_3$
o) $CH_2=CH-CH_2O-(C_3H_6O-)_{13}CH_3$
p) $C_4H_9O-(C_2H_4O-)_6(C_3H_6O-)_{25}H$

Hydrosilylierbare Olefine gemäß Anspruch:

| | |
|---|---|
| C6 = | Hexen-1 |
| C8 = | Octen-1 |
| C12 = | Dodecen-1 |
| C16 = | Hexadecen-1 |
| C20/24 = | Gemisch der α-Olefine mit 20 - 24 Kohlenstoffatomen, mittlere Molmasse = 301 |
| ST = | Styrol |
| AMS = | α-Methylstyrol |
| DCPD = | Dicyclopentadien |
| LIM = | Limonen |
| ALPH = | Allylphenol |
| VBC = | Vinylbenzylchlorid |

<u>Beispiel 1</u>

In einem mit Rührer, Thermometer, Gaseinleitung und Destillationsaufsatz versehenen Kolben werden 18,7 g (0,0188 Mol) des Polyethers c, 150,9 g (0,0375 Mol) des Polyethers f, 47,2 g (0,0313 Mol) des Polyethers m und 320 ml Toluol vorgelegt. Zur azeotropen Trocknung des Polyethergemisches werden 150 ml Toluol unter Stickstoffatmosphäre abdestilliert. Danach wird der Kolben mit einem Rückflußkühler versehen und weiter Stickstoff durch die Apparatur geleitet. Bei einer Temperatur von 105 °C werden 6,3 g (0,0375 Mol) Dodecen-1 und 55,6 g (0,1 Mol SiH) des Siloxans I dem Ansatz hinzugefügt. Nach gutem Durchmischen des Kolbeninhalts werden 0,18 g einer 10 %igen Lösung von $H_2PtCl_6{\cdot}6H_2O$ in i-Propanol zugeben. Man läßt den Ansatz 3,5 Std. abreagieren und erreicht einen SiH-Umsatz von 97,8 % (bestimmt über in alkalischem Medium mit n-Butanol abspaltbarem Wasserstoff). Der Ansatz wird mit 3 g Bentonit versetzt, 30 Min. gerührt und filtriert. Anschließend wird das Toluol bei 80 °C und 20 mbar abdestilliert. Man erhält ein klares, gelblich gefärbtes Produkt.

Beispiele 2 - 12

Entsprechend den Bedingungen von Beispiel 1 sind die folgenden Produkte hergestellt worden (siehe Tabelle). Als Katalysator wird $H_2PtCl_6{\cdot}6H_2O$ eingesetzt. Es werden ca. 0,7-g einer 10 %igen Lösung von $H_2PtCl_6{\cdot}6H_2O$ in i-Propanol pro 1000 g Ansatz (Siloxan- + Polyethermenge) verwendet. Als Lösungsmittel bei den Reaktionen werden 300 bis 800 ml Toluol pro 1000 g Ansatz eingesetzt.

| Beispiel | Siloxan | | | Polyether | | | Olefin | | | Umsatz in % |
|---|---|---|---|---|---|---|---|---|---|---|
| | Typ | g | Mol SiH | Typ | g | Mol | Typ | g | Mol | |
| 2 | II | 65,5 | 0,1 | a<br>f<br>m | 7,5<br>201,2<br>47,2 | 0,0125<br>0,05<br>0,0313 | C16 | 7,0 | 0,0313 | 99,5 |
| 3 | III | 60,5 | 0,1 | c<br>h<br>i<br>n | 12,5<br>37,8<br>27,6<br>75,6 | 0,0125<br>0,0125<br>0,0188<br>0,0375 | C6 | 3,7 | 0,0438 | 99,3 |
| 4 | IV | 69,2 | 0,1 | a<br>i<br>k<br>m | 7,5<br>27,6<br>50,1<br>75,5 | 0,0125<br>0,0188<br>0,0125<br>0,05 | C20 /<br>C24 | 9,4 | 0,0313 | 98,3 |
| 5 | III | 60,5 | 0,1 | c<br>g<br>i<br>o | 12,5<br>98,0<br>18,4<br>41,3 | 0,0125<br>0,025<br>0,0125<br>0,05 | ST | 2,6 | 0,025 | 97,4 |
| 6 | III | 60,5 | 0,1 | b<br>f<br>m | 7,3<br>201,2<br>56,6 | 0,0125<br>0,05<br>0,0375 | AMS | 3,0 | 0,025 | 98,9 |
| 7 | II | 65,5 | 0,1 | a<br>h<br>k<br>m | 7,5<br>56,7<br>75,2<br>66,1 | 0,0125<br>0,0188<br>0,0188<br>0,0438 | DCPD | 4,1 | 0,0313 | 97,1 |

| Beispiele | Siloxan | | | Polyether | | | Olefin | | | Umsatz in % |
|---|---|---|---|---|---|---|---|---|---|---|
| | Typ | g | Mol SiH | Typ | g | Mol | Typ | g | Mol | |
| 8 | III | 60,5 | 0,1 | a<br>f<br>m | 11,3<br>176,1<br>47,2 | 0,0188<br>0,0438<br>0,0313 | LIM | 4,3 | 0,0313 | 99,3 |
| 9 | III | 60,5 | 0,1 | a<br>f<br>m | 11,3<br>176,1<br>47,2 | 0,0188<br>0,0438<br>0,0313 | ALPH | 4,2 | 0,0313 | 98,7 |
| 10 | III | 60,5 | 0,1 | a<br>f<br>m | 11,3<br>176,1<br>47,2 | 0,0188<br>0,0438<br>0,0313 | VBC | 4,8 | 0,0313 | 98,9 |
| 11 | III | 60,5 | 0,1 | a<br>f<br>m | 7,5<br>150,9<br>37,8 | 0,0125<br>0,0375<br>0,025 | C12 | 8,4 | 0,05 | 99,1 |
| 12 | III | 60,5 | 0,1 | c<br>h<br>k<br>n | 12,5<br>56,7<br>50,1<br>37,8 | 0,0125<br>0,0188<br>0,0125<br>0,0188 | C8<br>LIM | 4,2<br>3,4 | 0,0375<br>0,025 | 97,9 |

Beispiel 13

In einem mit Rührer, Thermometer, Gaseinleitung und Destillationsaufsatz versehenen Kolben werden 13,2 g (0,022 Mol) des Polyethers d, 87,9 g (0,022 Mol) des Polyethers e, 66,0 g (0,022 Mol) des Polyethers l, 78,7 g (0,044 Mol) des Polyethers p und 1100 ml Toluol vorgelegt. Unter Stickstoffabdeckung werden 150 ml Toluol zur azeotropen Trocknung des Polyethergemisches abdestilliert. Bei 50 °C wird der Destillationsaufsatz gegen einen Rückflußkühler ausgetauscht. Anschließend werden 83 g (0,1 Mol SiX) des Siloxans V zugesetzt. Dann wird bei 60 °C Ammoniakgas eingeleitet bis der Kolbeninhalt ammoniakalisch reagiert. Man läßt noch eine weitere Stunde unter schwachem Einleiten von Ammoniakgas nachreagieren. Anschließend wird das ausgefallene Salz abfiltriert. Danach wird bei 70 °C und 20 mbar Toluol abdestilliert. Man erhält ein hellbraunes, fast klares Produkt.

Beispiel 14
(nicht erfindungsgemäß)

Unter den Reaktionsbedingungen der Beispiele 2 - 12 werden 7,5 g (0,0125 Mol) des Polyethers a, 301,8 g (0,075 Mol) des Polyethers f und 56,6 g (0,0375 Mol) des Polyethers m mit 65,5 g (0,1 Mol SiH) des Siloxans II umgesetzt. Der SiH-Umsatz beträgt 99,4 %.

Anwendungstechnische Prüfung

In vergleichenden Versuchen mit Konditioniershampoos an Strähnen aus indoeuropäischem Humanhaar werden die oben beschriebenen Eigenschaften gefunden:
Dazu werden jeweils

| | |
|---|---|
| 2,0 % | des zu testenden Polyethersiloxans mit |
| 3,0 % | TEGOSOFT GC (Glycerincocoate mit 7 Mol Ethylenoxid) |
| 40,0 % | Texapon N 25 (Laurylethersulfat) |
| 43,2 % | Wasser |
| 10,0 % | TEGO Betain F 50 (Cocosamidopropylbetain) |
| 1,3 % | ANTIL 171 (Verdickungsmittel) |
| 0,5 % | NaCl |

zu einer Shampooformulierung abgemischt.

In dem Vergleichstest werden Noten von null bis vier vergeben, wobei null die schlechteste (mangelhaft) und vier die beste (sehr gut) Bewertung ist. Bei Vergleich der sieben Shampoos miteinander werden die folgenden Noten, gemittelt über zehn Probanden, erhalten:

| | Produkt nach Beispiel | | | | | | ABIL B 8852 |
|---|---|---|---|---|---|---|---|
| | 2 | 3 | 4 | 8 | 12 | 14 | |
| Im nassen Haar | | | | | | | |
| Verknotung | 2,5 | 2,3 | 2,5 | 2,4 | 2,5 | 2,2 | 2,2 |
| Kämmbarkeit | 2,2 | 2,2 | 2,3 | 2,3 | 2,2 | 1,9 | 2,2 |
| Griff | 2,2 | 2,2 | 2,3 | 2,2 | 2,3 | 2,2 | 2,2 |
| Im getrockneten Haar | | | | | | | |
| Kämmbarkeit | 3,0 | 3,0 | 3,1 | 3,0 | 3,1 | 2,8 | 2,8 |
| Griff | 2,6 | 2,6 | 2,8 | 2,7 | 2,6 | 1,8 | 1,8 |
| Glanz | 2,2 | 2,1 | 2,2 | 2,2 | 2,2 | 2,0 | 2,0 |

Die Produkte der Beispiele 2, 3, 4, 8, und 12 sind erfindungsgemäß.

Das Produkt nach Beispiel 14 und ABIL B 8852 (Handelsprodukt der Th. Goldschmidt AG) sind nicht erfindungsgemäß.

In diesem Vergleich wird besonders der Griff der trockenen Haare, behandelt mit den Shampoos der erfindungsgemäßen Verbindungen der Beispiele 2, 3, 4, 8, und 12, besser bewertet als bei den Vergleichsbeispielen mit den nicht-erfindungsgemäßen Produkten. Es wird auch die Verknotung und die Kämmbarkeit der nassen Haare nach der

Behandlung mit einem Shampoo auf Basis der erfindungsgemäßen Verbindungen etwas besser bewertet. Das gleiche gilt für die Kämmbarkeit der getrockneten Haare.

## Patentansprüche

1. Polysiloxan-Polyoxyalkylen-Blockmischpolymerisat der allgemeinen Formel

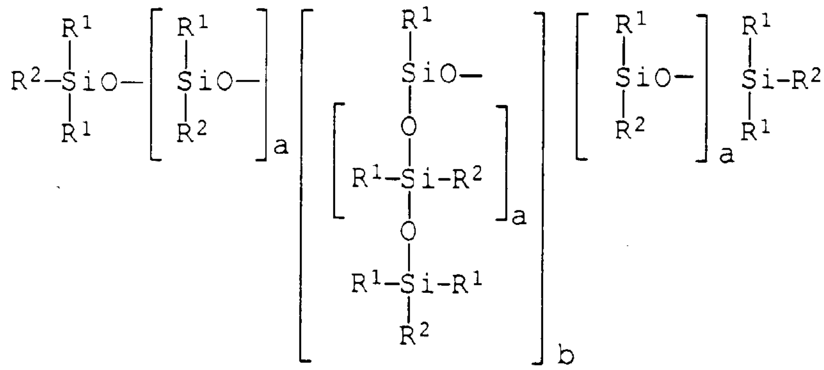

wobei die Reste

$R^1$ Alkylreste mit 1 bis 4 Kohlenstoffatomen oder Phenylreste sind, jedoch mindestens 90 % der Reste $R^1$ Methylreste sind,

$R^2$ (1) den Resten $R^1$ entsprechen oder
(2) durch Addition von Kohlenwasserstoffen mit einer olefinischen Doppelbindung an SiH-Gruppen des Siloxans erhaltene Reste mit 6 bis 30 Kohlenstoffatomen oder
(3) $-M-R^3$ Reste sind, wobei

M ein zweiwertiger Rest der Formel $-R^4{}_xO-$ ist, in dem $R^4$ ein zweiwertiger Alkylenrest, der auch verzweigt sein kann, ist und x einen Wert von 0 oder 1 hat,

$R^3$ ein Gemisch von Polyetherresten, enthaltend mindestens einen

(1) Polyoxyalkylenrest A mit einem mittleren Molgewicht von 600 bis 5500, der aus 20 bis 100 Gew.-% Oxyethyleneinheiten und 80 bis 0 Gew.-% Oxypropyleneinheiten besteht, und einen
(2) Polyoxyalkylenrest B mit einem mittleren Molgewicht von 700 bis 5000, der aus 0 bis < 20 Gew.-% Oxyethyleneinheiten und 100 bis 80 Gew.-% Oxypropyleneinheiten besteht,

wobei jeweils bis zu 20 Gew.-% der Oxypropyleneinheiten durch Oxybutyleneinheiten ersetzt sein können, und
wobei das Molverhältnis der Polyoxyalkylenreste A zu den Polyoxyalkylenresten B 1 : 4 bis 4 : 1 beträgt,

mit der Maßgabe, daß

(I) die Anzahl der Reste $R^2$ mit der Bedeutung (2) mindestens gleich 1 und höchstens 30 % des Zahlenwertes der Anzahl der Siliciumatome ist und

(II) im durchschnittlichen Blockmischpolymerisat mindestens zwei Reste $R^3$ vorhanden sind,

b einen Wert von 0 bis 10 hat,
a einen Wert von 10 bis 100 hat, wenn b = 0 ist oder einen Wert von 3 bis 70 hat, wenn b > 0 und ≤ 4 ist, oder einen Wert von 3 bis 30 hat, wenn b > 4 ist.

2. Blockmischpolymerisat nach Anspruch 1, dadurch gekennzeichnet, daß die Reste $R^2$ mit der Bedeutung (2) Reste

der Formel

$-(CH_2)_{5-29}-CH_3$ 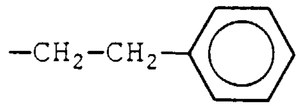 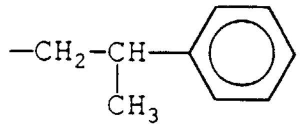

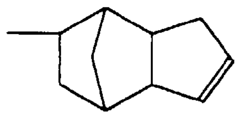 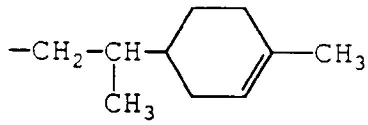

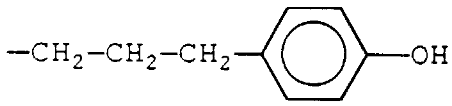 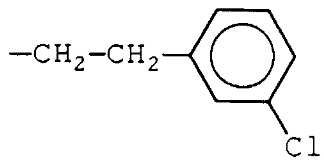

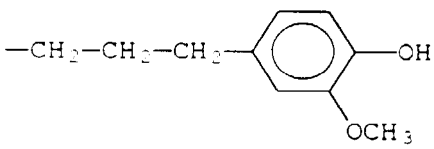 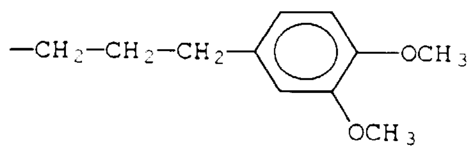

sind.

**3.** Blockmischpolymerisat nach Anspruch 1 oder 2, dadurch gekennzeichnet, daß die Polyoxyalkylenblöcke der Formel $(C_mH_{2m}O-)_nR^5$ entsprechen, wobei die Indices n und m so gewählt sind, daß die Bedingungen hinsichtlich der Zusammensetzung und des jeweiligen Molgewichtes der verschiedenen Polyoxyalkylenblöcke erfüllt sind, und $R^5$ ein Alkylrest mit 1 bis 4 Kohlenstoffatomen, ein Acylrest oder ein -O-CO-NH-$R^6$ Rest, wobei $R^6$ einen Alkyl- oder Arylrest bedeutet, ist.

**4.** Verfahren zur Herstellung der Blockmischpolymerisate nach einem oder mehreren der vorhergehenden Ansprüche, wobei x einen Wert von 1 hat, dadurch gekennzeichnet, daß man an Organopolysiloxane der allgemeinen Formel

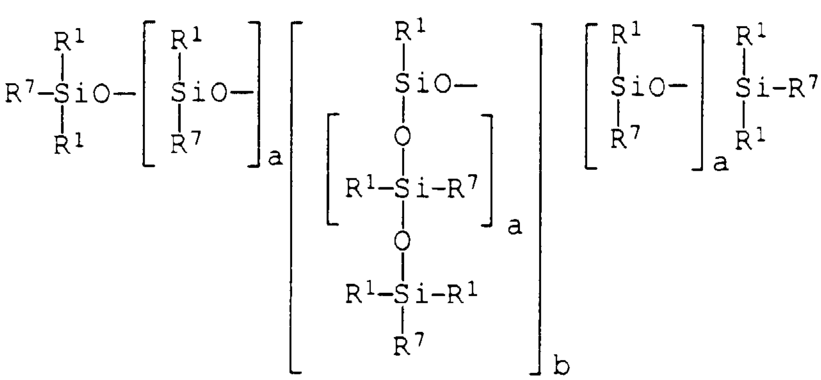

wobei die Reste

$R^1$ und die Indices a und b die bereits angegebene Bedeutung haben,
$R^7$ (1) den Resten $R^1$ entsprechen oder
(2) SiH-Reste sind,

mit der Maßgabe, daß mindestens 3 Reste $R^7$ SiH-Reste sind,

a) Kohlenwasserstoffe mit 6 bis 30 Kohlenstoffatomen und einer olefinischen Doppelbindung und
b) ein Gemisch von Polyethern der Formel

$$CH_2{=}CH\text{-}R^8\text{-}A \text{ und } CH_2{=}CH\text{-}R^8\text{-}B$$

wobei der Rest $R^8$ dem um die Gruppe $-(CH_2)_2-$ verminderten Rest $R^4$ entspricht und die Polyether im Molverhältnis 1 : 4 bis 4 : 1 vorliegen,

in Gegenwart von für die Hydrosilylierung geeigneten Platinkatalysatoren nacheinander oder gleichzeitig hydrosilylierend addiert.

**5.** Verfahren zur Herstellung der Blockmischpolymerisate nach einem oder mehreren der vorhergehenden Ansprüche, wobei x einen Wert von 0 hat, dadurch gekennzeichnet, daß man Organopolysiloxane der allgemeinen Formel

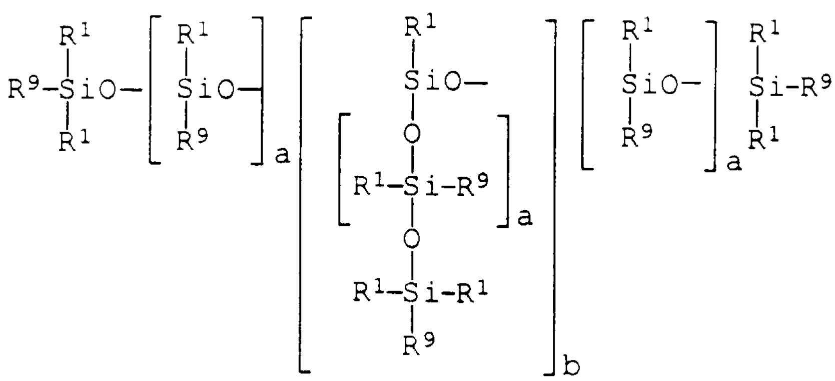

wobei die Reste

$R^1$ und die Indices a und b die bereits angegebene Bedeutung haben,
$R^9$ (1) den Resten $R^1$ entsprechen oder
(2) durch Addition von Kohlenwasserstoffen mit einer olefinischen Doppelbindung an SiH-Gruppen des Siloxans erhaltene Reste mit 6 bis 30 Kohlenstoffatomen oder
(3) Halogen- oder sonstige saure Reste, wie $-SO_{4/2}$ oder $-CH_3SO_3$ sind,

mit der Maßgabe, daß mindestens 1 Rest $R^9$ die Bedeutung (2) und mindestens 2 Reste $R^9$ die Bedeutung (3) haben,
mit einem Gemisch der Polyether

$$A\text{—}OH \text{ und } B\text{—}OH ,$$

welche in einem Molverhältnis von 1 : 4 bis 4 : 1 vorliegen, mit einer solchen Menge des Polyethergemisches A-OH und B-OH, daß im gewünschten Organopolysiloxan mindestens 2 Polyetherreste gebunden sind, in Gegenwart eines Säureacceptors bei Temperaturen von ≥ 50°C umsetzt.

**6.** Verwendung der Blockmischpolymerisate nach Anspruch 1, 2 oder 3 als Zusatzmittel für Haarkosmetika, insbesondere für Haarshampoos, in Mengen von 0,5 bis 4 Gew.-%, bezogen auf Gesamtmenge des Shampoos, zur Verbesserung des Griffes und der Kämmbarkeit der Haare.

## Claims

1. Polysiloxane-polyoxyalkylene block copolymer of the general formula

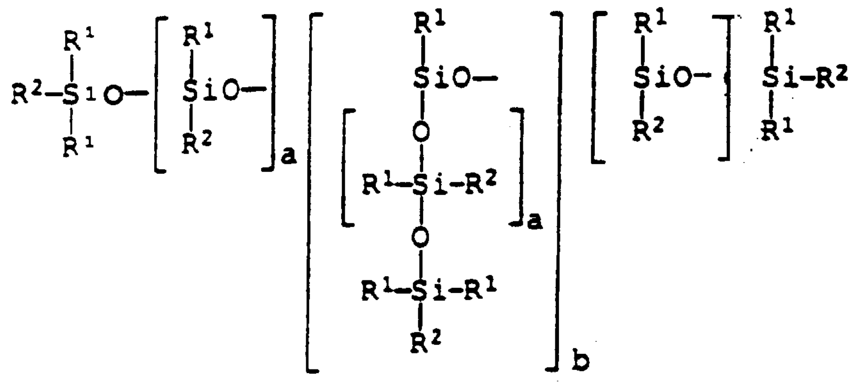

where the radicals

$R^1$ are alkyl radicals having from 1 to 4 carbon atoms or phenyl radicals, but at least 90% of the radicals $R^1$ are methyl radicals,

$R_2$ (1) correspond to the radicals $R^1$ or
(2) are radicals having from 6 to 30 carbon atoms obtained by addition of hydrocarbons having an olefinic double bond onto SiH groups of the siloxane or
(3) are $-M-R^3$ radicals, where

M is a divalent radical of the formula $-R^4{}_xO-$, where $R^4$ is a divalent alkylene radical which may also be branched and x is 0 or 1,

$R^3$ is a mixture of polyether radicals containing at least one

(1) polyoxyalkylene radical A having a mean molecular weight of from 600 to 5500 and comprising from 20 to 100% by weight of oxyethylene units and from 80 to 0% by weight of oxypropylene units, and a
(2) polyoxyalkylene radical B having a mean molecular weight of from 700 to 5000 and comprising from 0 to < 20% by weight of oxyethylene units and from 100 to 80% by weight of oxypropylene units,

where in each case up to 20% by weight of the oxypropylene units can be replaced by oxybutylene units, and where the molar ratio of the polyoxyalkylene radicals A to the polyoxyalkylene radicals B is from 1:4 to 4:1,

with the proviso that

(I) the number of radicals $R^2$ having the meaning (2) is at least 1 and at most 30% of the number of silicon atoms and
(II) at least two radicals $R^3$ are present in the average block copolymer,

b is from 0 to 10,
a is from 10 to 100 when b = 0 or is from 3 to 70 when b > 0 and ≤ 4 or is from 3 to 30 when b > 4.

2. Block copolymer according to Claim 1, characterized in that the radicals $R^2$ having the meaning (2) are radicals of the formula

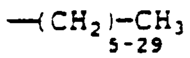 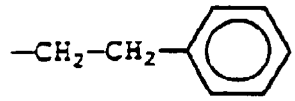 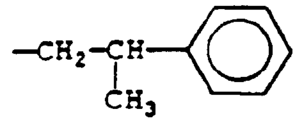

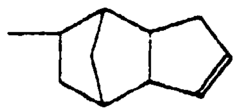 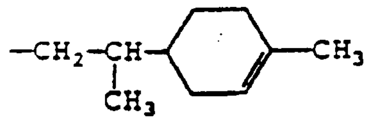

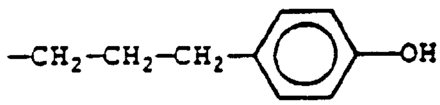 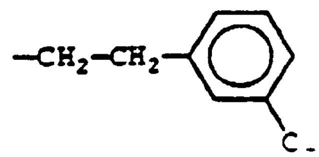

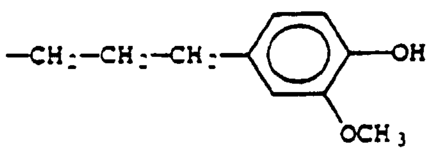 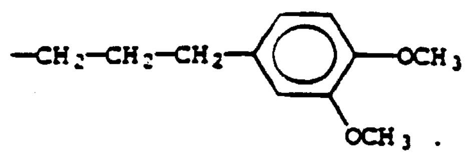 .

**3.** Block copolymer according to Claim 1 or 2, characterized in that the polyoxyalkylene blocks correspond to the formula $(C_mH_{2m}O-)_nR^5$, where the indices n and m are selected such that the conditions in respect of the composition and the respective molecular weight of the various polyoxyalkylene blocks are met, and $R^5$ is an alkyl radical having from 1 to 4 carbon atoms, an acyl radical or an $-O-CO-NH-R^6$ radical, where $R^6$ is an alkyl or aryl radical.

**4.** Process for preparing the block copolymers according to one or more of the preceding claims, where x is 1, characterized in that

a) hydrocarbons having from 6 to 30 carbon atoms and an olefinic double bond and
b) a mixture of polyethers of the formulae

$$CH_2=CH-R^8-A \text{ and } CH_2=CH-R^8-B$$

where the radical $R^8$ corresponds to the radical $R^4$ decreased by the group $-(CH_2)_2-$ and the polyethers are present in a molar ratio of from 1:4 to 4:1, are added in succession or simultaneously in a hydrosilylation reaction onto organopolysiloxanes of the general formula

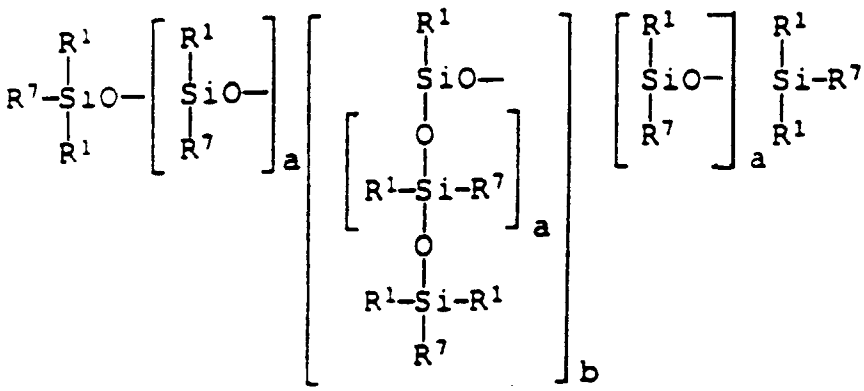

where the radicals

$R^1$ and the indices a and b are as defined above,
$R^7$ (1) correspond to the radicals $R^1$ or (2) are SiH radicals,

with the proviso that at least 3 radicals $R^7$ are SiH radicals, in the presence of platinum catalysts suitable for hydrosilylation.

**5.** Process for preparing the block copolymers according to one or more of the preceding claims, where x is 0, characterized in that organopolysiloxanes of the general formula

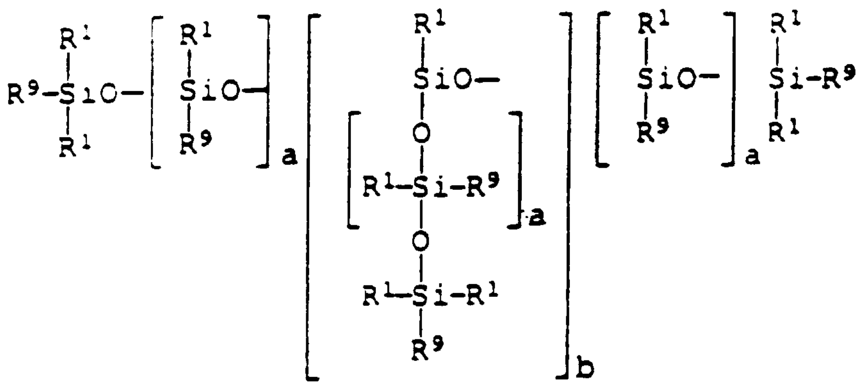

where the radicals

$R^1$ and the indices a and b are as defined above,
$R^9$ (1) correspond to the radicals $R^1$ or
(2) are radicals having from 6 to 30 carbon atoms obtained by addition of hydrocarbons having an olefinic double bond onto SiH groups of the siloxane or
(3) halogen radicals or other acid radicals such as $-SO_{4/2}$ or $-CH_3SO_3$,

with the proviso that at least 1 radical $R^9$ has the meaning (2) and at least 2 radicals $R^9$ have the meaning (3), are reacted with a mixture of the polyethers

A-OH and B-OH

which are present in a molar ratio of from 1:4 to 4:1, where the amount of the polyether mixture of A-OH and B-OH is such that at least 2 polyether radicals are present in bound form in the desired organopolysiloxane, in the

presence of an acid acceptor at temperatures of ≥ 50°C.

6. Use of the block copolymers according to Claim 1, 2 or 3 as additives for hair cosmetics, in particular for hair shampoos, in amounts of from 0.5 to 4% by weight, based on the total amount of shampoo, for improving the feel and the combability of the hair.

**Revendications**

1. Copolymère séquencé de polysiloxane et polyoxyalkylène de formule générale :

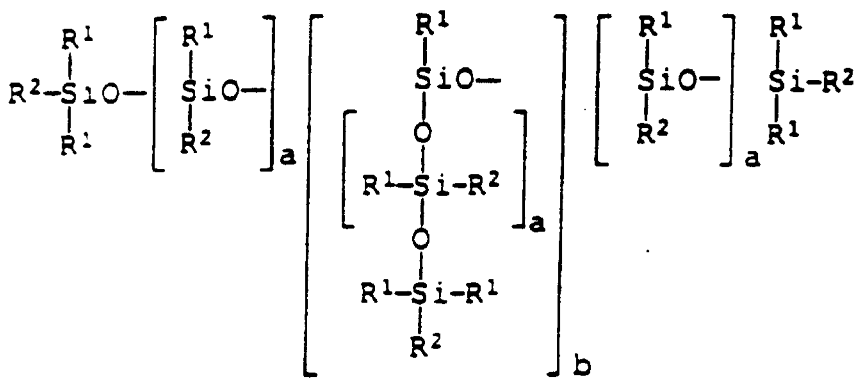

dans laquelle :

les radicaux $R^1$ sont des radicaux alcoyle avec 1 à 4 atomes de carbone ou des radicaux phényle, 90% au moins des radicaux $R^1$ étant toutefois des radicaux méthyle;
les radicaux $R^2$ :

(1) correspondent aux radicaux $R^1$ ou
(2) sont des radicaux, obtenus par addition d'hydrocarbures, ayant une double liaison oléfinique, sur des groupes SiH du siloxane, avec 6 à 30 atomes de carbone, ou
(3) des radicaux $-M-R^3$, dans lesquels :

M est un radical bivalent de formule $-R^4_xO-$, dans lequel $R^4$ est un radical alkylène bivalent, qui peut également être ramifié, et x a une valeur de 0 à 1, et
$R^3$ est un mélange de radicaux polyéther, contenant au moins :

(1) un radical polyoxyalkylène A ayant un poids moléculaire moyen de 600 à 5500, qui est composé de 20 à 100% en poids d'unités oxyéthylène et de 80 à 0% en poids d'unités oxypropylène, et
(2) un radical polyoxyalkylène B ayant un poids moléculaire moyen de 700 à 5000, qui est composé de 0 à <20% en poids d'unités oxyéthylène et de 100 à 80% en poids d'unités oxypropylène,

jusqu'à 20% en poids des unités oxypropylène pouvant dans chaque cas être remplacées par des unités oxybutylène, et
le rapport molaire des radicaux polyoxyalkylène A aux radicaux polyoxyalkylène B valant 1:4 à 4:1,

sous réserve que
(I) le nombre des radicaux $R^2$ ayant la signification (2) soit au moins égal à 1 et au plus à 30% de la valeur du nombre des atomes de silicium, et que
(II) au moins deux radicaux $R^3$ soient présents dans le copolymère séquencé moyen;

b vaut de 0 à 10, et
a vaut de 10 à 100, lorsque b = 0, ou de 3 à 70, lorsque b > 0 et ≤ 4, ou de 3 à 30, lorsque b > 4.

**2.** Copolymère séquencé suivant la revendication 1, caractérisé en ce que les radicaux $R^2$ ayant la signification (2) sont des radicaux de formule :

$-(CH_2)_{5-29}-CH_3$ 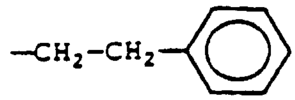 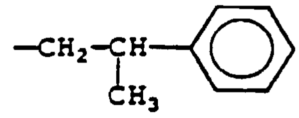

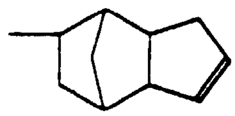 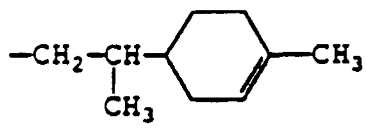

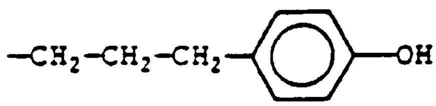 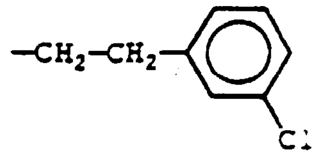

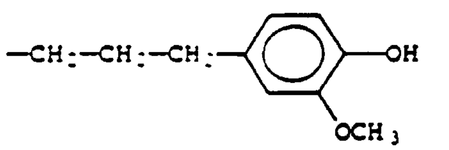 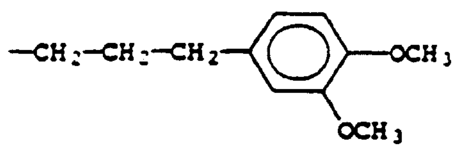

**3.** Copolymère séquencé suivant la revendication 1 ou 2, caractérisé en ce que les blocs polyoxyalkylène correspondent à la formule $(C_mH_{2m}O-)_nR^5$, les indices n et m étant choisis de telle sorte que les conditions relatives à la composition et au poids moléculaire respectif des différents blocs de polyoxyalkylène soient satisfaites, et en ce que $R^5$ est un radical alcoyle ayant 1 à 4 atomes de carbone, un radical acyle ou un radical -O-CO-NH-$R^6$, $R^6$ représentant un radical alcoyle ou un radical aryle.

**4.** Procédé de préparation des copolymères séquencés suivant l'une ou plusieurs des revendications précédentes, dans lequel x vaut 1, caractérisé en ce que l'on ajoute, de manière hydrosilylante l'un après l'autre ou simultanément, à des organopolysiloxanes de formule générale :

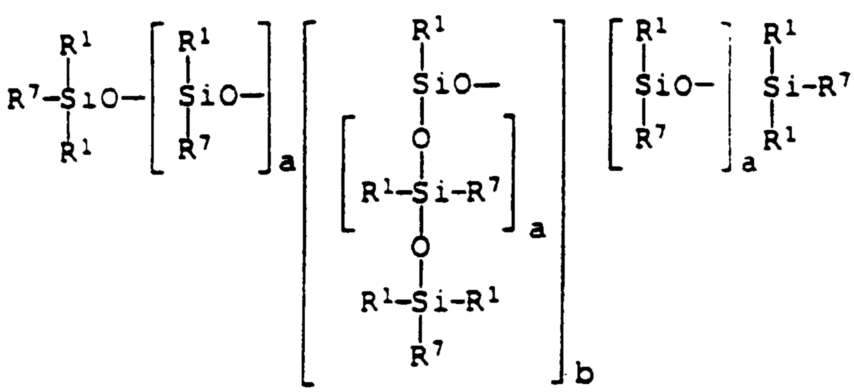

dans laquelle :
les radicaux $R^1$ et les indices a et b ont les significations déjà données, et

les radicaux $R^7$ :

(1) correspondent aux radicaux $R^1$, ou
(2) sont des radicaux SiH,

sous réserve qu'au moins 3 radicaux $R^7$ soient des radicaux SiH,

(a) des hydrocarbures ayant 6 à 30 atomes de carbone et une double liaison oléfinique, et
(b) un mélange de polyéthers de formules :

$$CH_2{=}CH\text{-}R^8\text{-}A \text{ et } CH_2{=}CH\text{-}R^8\text{-}B$$

dans lesquelles le radical $R^8$ correspond au radical $R^4$ diminué du groupe $-(CH_2)_2-$, et dans lesquelles les polyéthers sont présents dans un rapport molaire de 1:4 à 4:1,
en présence de catalyseurs au platine convenant à une hydrosilylation.

**5.** Procédé de préparation des copolymères séquencés suivant l'une ou plusieurs des revendications précédentes, dans lequel x vaut 0, caractérisé en ce que des organopolysiloxanes de formule générale :

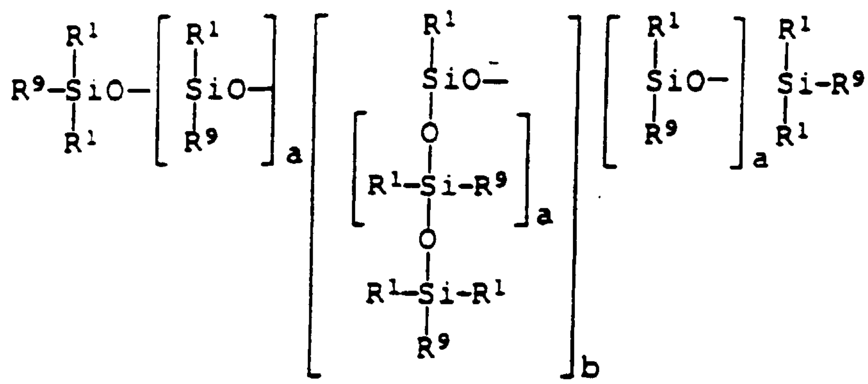

dans laquelle :
les radicaux $R^1$ et les indices a et b ont les significations déjà données, et
les radicaux $R^9$ :

(1) correspondent aux radicaux $R^1$, ou
(2) sont des radicaux ayant 6 à 30 atomes de carbone obtenus par addition d'hydrocarbures ayant une double liaison oléfinique sur les groupes SiH du siloxane, ou
(3) sont des halogènes ou d'autres radicaux acides, comme $-SO_{4/2}$ ou $-CH_3SO_3$,

sous réserve qu'au moins 1 radical $R^9$ ait la signification (2) et qu'au moins 2 radicaux $R^9$ aient la signification (3), avec un mélange des polyéthers

A-OH et B-OH,

qui se trouvent dans un rapport molaire de 1:4 à 4:1, sont mis à réagir avec une quantité du mélange de polyéthers A-OH et B-OH telle qu'au moins 2 radicaux polyéther sont liés dans l'organopolysiloxane soulaité, en présence d'un accepteur d'acide, à des températures ≥ 50°C.

**6.** Utilisation des copolymères séquencés suivant la revendication 1, 2 ou 3 comme additifs pour des produits capillaires, en particulier pour des shampoings capillaires, en des quantités de 0,5 à 4% en poids, par rapport au poids total du shampoing, pour l'amélioration du maintien et du coiffage des cheveux.